# FASCICULE DE BREVET EUROPEEN

(11) **EP 2 528 583 B1**
(45) Date de publication et mention de la délivrance du brevet: **20.07.2016**
(21) Numéro de dépôt: 11701155.1
(22) Date de dépôt: 31.01.2011
(51) Int. Cl.: C11B 1/10, C11C 3/00, B01D 11/04, C11C 1/02

(54) **EXTRACTION LIQUIDE / LIQUIDE**
FLÜSSIG-FLÜSSIG-EXTRAKTION
LIQUID/LIQUID EXTRACTION

(30) Priorité: 28.01.2011 FR 1150682; 29.01.2010 FR 1050644
(43) Date de publication de la demande: 05.12.2012
(73) Titulaire: Laboratoires Expanscience, 92400 Courbevoie (FR)
(72) Inventeur: SAUNOIS, Alex, F-28210 Nogent-le-Roi (FR); LEGRAND, Jacques, F-61290 Neuilly-sur-Eure (FR); MERCIER, Eglantine, F-78120 Rambouillet (FR)
(74) Mandataire: Regimbeau
(86) Numéro de dépôt international: PCT/EP2011/051321
(87) Numéro de publication internationale: WO 2011/092329

(56) Documents cités:
- DE-A1- 19 608 463
- FR-A1- 2 678 632
- FR-A1- 2 803 598
- JP-A- 7 291 890
- US-A- 5 403 514
- US-A- 5 458 692
- US-A1- 2004 018 258
- US-A1- 2005 209 468
- US-A1- 2006 086 664
- US-A1- 2006 287 533
- US-B2- 6 890 425

## Description

La présente invention concerne un procédé d'extraction de fractions insaponifiables, notamment partielles ou totales, d'huiles ou de beurres végétaux ou d'huiles provenant de micro-organismes.

Les insaponifiables ou fractions insaponifiables d'un corps gras sont constitués de composés qui forment la partie d'un corps gras qui, après action prolongée d'une base alcaline, reste insoluble dans l'eau et peut être extraite par un solvant organique.

La plupart des insaponifiables d'huiles ou de beurres végétaux comprennent plusieurs grandes familles de substances. Parmi ces grandes familles on peut citer les hydrocarbures saturés ou insaturés, les alcools aliphatiques ou terpéniques, les stérols, les tocophérols, les pigments caroténoïdes, xanthophiles, ainsi que des familles spécifiques, notamment une ou deux, dans le cas de certaines huiles et beurres.

Les procédés usuels d'obtention des insaponifiables des huiles végétales et des beurres végétaux visent à extraire tout ou partie des grandes familles qui les composent, permettant de préparer les fractions partielles ou totales des insaponifiables.

Les fractions partielles ou totales d'insaponifiables sont notamment recherchées pour leurs propriétés pharmacologiques, cosmétiques et nutritionnelles.

Les procédés usuels d'obtention des insaponifiables des huiles végétales et des beurres végétaux comprennent une étape de saponification de la matière grasse et une extraction du produit cible (l'insaponifiable) par un solvant organique.

Les solvants les plus communément utilisés sont les solvants des huiles, tels que les alcanes (hexane, heptane,...) et les solvants chlorés (1,2-dichloroéthane ou DCE, trichloroéthane, 1-chlorobutane, tétrachlorure de carbone,...). Parmi ces derniers, le DCE et le 1-chlorobutane constituent les meilleurs candidats, notamment au regard de leur rendement d'extraction et de leur sélectivité.

FR 2 803 598 décrit un procédé d'extraction des insaponifiables d'huile végétale comprenant une étape d'extraction par un solvant organique, notamment le chloro-1-butane.

Cependant, sur le plan industriel, la toxicité du solvant mis en oeuvre ainsi que sa stabilité chimique doivent être pris en compte. A ce titre, les solvants chlorés, et en particulier le 1,2-dichloroéthane (DCE) et le 1-chlorobutane, présentent trois inconvénients majeurs : ils peuvent se dégrader en milieu basique (ce qui est le cas des solutions savonneuses de saponification), ils sont classés parmi les solvants toxiques, notamment CMR pour le DCE, et ils provoquent des impacts négatifs sur l'environnement.

Les solvants chlorés présentent fréquemment une toxicité et/ou une dangerosité indésirable(s).

Par ailleurs, tant d'un point de vue économique que d'un point de vue environnemental, les procédés d'obtention de fractions insaponifiables peuvent requérir l'utilisation de quantités de solvants organiques non adaptées pour la viabilité du procédé, présenter un nombre d'étapes d'extraction insatisfaisants, être trop lents et/ou présenter des séparations de phase insatisfaisantes, par exemple en provoquant une émulsion non désirée. En particulier, il peut être nécessaire ou utile d'ajuster la concentration de la matière grasse mise en oeuvre lors de la saponification pour optimiser le rapport de solvant utilisé.

La présente invention vise donc à résoudre en tout ou partie les problèmes évoqués ci-dessus. En particulier, l'invention vise à fournir un procédé plus économique, plus direct, plus amical pour l'environnement, nécessitant une quantité de solvant organique plus faible, plus facile à mettre en oeuvre, plus rapide, générant des conditions moins toxiques et/ou moins dangereuses, améliorant la séparation de phases, permettant l'obtention d'insaponifiables avec un rendement, un coût et/ou une sélectivité au moins comparables aux procédés déjà existants.

En particulier, il est souhaitable que le(s) solvant(s) impliqué(s) soi(en)t moins dangereux, moins toxique(s), notamment non-classé(s) CMR, soi(en)t chimiquement plus stable(s) que le 1,2-dichloroéthane et/ou que le 1-chlorobutane et/ou permette(nt) d'extraire les insaponifiables avec un rendement et/ou une sélectivité au moins comparables aux rendements et sélectivités obtenus en utilisant le 1,2-dichloroéthane et/ou le 1-chlorobutane.

Les solvants dits « classés CMR » peuvent être ceux qui sont présentés dans la liste en annexes de la directive 2009/2/CE du 15 janvier 2009, notamment disponible à l'adresse http://eur-lex.europa.eu/LexUriServ/LexUriServ.do?uri=OJ:L:2009:011:0006:0082:FR:PDF, cette première liste étant appelée ci-après « liste CMR UE1 », ceux listés dans la Classification européenne réglementaire des produits chimiques cancérogènes, mutagènes et toxiques pour la reproduction - 31e ATP, 2009, notamment disponible à l'adresse http://www.prc.cnrs-gif.fr/en_telechargement/cmr31.pdf cette deuxième liste étant appelée ci-après « liste CMR UE2 », et/ou ceux listés dans la liste « Chemicals known or suspected to cause cancer or reproductive toxicity » du 1^{er} septembre 2009 établie par le « California department of public health, occupational health branch, California safe cosmetic program » liée à la « California Safe Cosmetics Act of 2005 » cette troisième liste étant appelée ci-après « liste CMR US ».

Lorsque dans le présent texte est utilisé l'expression liste CMR UE, on entend liste CMR UE1 et/ou CMR UE2.

Un des objectifs plus particulier est d'obtenir une fraction insaponifiable spécifique, par exemple ayant une teneur plus forte en certains composés et moins forte en d'autres, en particulier comprenant uniquement une ou certaines des familles de composés de l'insaponifiable total, et/ou une fraction ayant une composition comparable à celle obtenue par un procédé impliquant du DCE et/ou du 1-chlorobutane.

Les insaponifiables peuvent être composés de nombreux constituants, en particulier comprenant les grandes familles de substances définies ci-dessus et/ou des familles spécifiques. Il peut être souhaitable d'extraire le plus complètement possible au moins une de ces familles, notamment au moins deux, en particulier au moins trois, tout particulièrement au moins quatre, voire au moins cinq, tout particulièrement au moins six, et encore plus particulièrement toutes les familles composant l'insaponifiable d'une huile ou d'un beurre donné.

Autrement exprimé, le procédé selon l'invention vise soit à permettre l'obtention d'une fraction partielle spécifique de l'insaponifiable, notamment présentant une teneur enrichie en au moins une des familles composant l'insaponifiable, voire d'extraire spécifiquement un ou plusieurs composés particulier de l'insaponifiable, soit à permettre d'obtenir la fraction insaponifiable dite totale ou quasi-totale.

La présente invention a ainsi pour objet un procédé d'extraction d'une fraction insaponifiable, notamment partielle ou totale, contenue dans une huile ou un beurre végétal, dans une huile provenant d'un micro-organisme, dans un concentrât d'huile ou de beurre végétal ou d'huile provenant d'un micro-organisme, ou dans un co-produit de l'industrie du raffinage des huiles végétales ou des huiles provenant de micro-organismes, tel que les échappées de désodorisation, comprenant au moins :
A) une étape de transformation par saponifications ou les estérifications desdites huiles, dudit beurre ou dudit co-produit de l'industrie du raffinage des huiles végétales ou des huiles provenant de micro-organismes en solution hydro-alcoolique,
B) une étape d'extraction de la solution hydro-alcoolique dans laquelle la fraction grasse est séparée de la fraction insaponifiable par une extraction liquide / liquide ou par une distillation,
C) éventuellement, une étape de purification de l'insaponifiable choisie dans le groupe constitué par les cristallisations et les extractions liquide / liquide,
ledit procédé étant caractérisé en ce qu'au moins une étape parmi les extractions liquide / liquide de l'étape B, les cristallisations de l'étape C et les extractions liquide / liquide de l'étape C est effectuée en utilisant un premier système de solvants comprenant une teneur en tert-butyl éthers, notamment en 2-éthoxy-2-méthylpropane, encore appelé éthyl-tert-butyl-éther (ETBE), ou un mélange de tert-butyl éthers avec des solvants aromatiques fluorés, notamment le trifluorotoluène (BTF) et l'hexafluorobenzène (BHF), des solvants comprenant au moins un atome de silicium, notamment l'hexaméthyldisiloxane (HMDS) et le tétraméthylsilane (TMS) ou le méthyl-tétrahydrofurane (MeTHF), et leurs mélanges d'au moins 50% en volume par rapport au volume total du système de solvants.

Les numéros CAS de ces différents solvants étant les suivants BTF : 98-08-8 ; BHF : 392-56-3 ; ETBE : 37-92-3 ; MTBE : 1634-04-4 ; HMDS : 107-46-0 ; TMS : 75-76-3 ; et MeTHF : 96-47-9.

Par « fraction totale », on entend au sens de la présente invention le fait que cette fraction comprend toutes les familles de substances composant l'insaponifiable présentes dans l'huile végétale ou dans l'huile provenant d'un micro-organisme ou dans le beurre végétal considéré.

Par « fraction partielle », on entend au sens de la présente invention le fait que cette fraction comprend au moins une des familles de substances composant l'insaponifiable présentes dans l'huile ou le beurre végétal ou l'huile provenant d'un micro-organisme considéré.

Selon un mode de réalisation particulier, l'invention concerne un procédé dans lequel l'étape B) comprend, ou consiste en, une extraction liquide / liquide avec le premier système de solvants.

Selon un autre mode de réalisation particulier, l'invention concerne un procédé dans lequel l'étape C) comprend, ou consiste en, une cristallisation ou une extraction liquide / liquide avec le premier système de solvants.

Selon un mode de réalisation encore plus particulier, le procédé comprend une étape B) comprenant, ou consistant en, une extraction liquide / liquide avec un premier système de solvants et une étape C) comprenant ou consistant en une cristallisation et/ou une extraction liquide / liquide avec un premier système de solvants identique ou différent de celui utilisé en étape A).

Tout particulièrement, l'étape C) peut permettre de purifier la fraction insaponifiable, d'enrichir celle-ci en une ou plusieurs familles de substances composant l'insaponifiable présente(s) dans l'huile végétale, l'huile provenant d'un micro-organisme ou le beurre végétal considéré. En particulier cela peut permettre d'isoler une fraction spécifique de l'insaponifiable de soja, tels que les composés stéroliques, ou de l'insaponifiable d'avocat, tels que les composés furaniques et/ou stéroliques.

Le premier système de solvants peut comprendre une teneur en tert-butyl éthers, notamment en 2-éthoxy-2-méthylpropane, encore appelé éthyl-tert-butyl-éther (ETBE), ou un mélange de tert-butyl éthers avec des solvants aromatiques fluorés, notamment le trifluorotoluène (BTF) et l'hexafluorobenzène (BHF), des solvants comprenant au moins un atome de silicium, notamment l'hexaméthyldisiloxane (HMDS) et le tétraméthylsilane (TMS) ou le méthyl-tétrahydrofurane (MeTHF), et leurs mélanges d'au moins 60 %, notamment au moins 75%, en particulier au moins 90%, plus particulièrement au moins 95%, encore plus particulièrement au moins 99% en volume, par rapport au volume total du premier système de solvants.

En particulier, le premier système de solvants est constitué de tert-butyl éther, notamment le 2-éthoxy-2-méthylpropane, encore appelé éthyl-tert-butyl-éther (ETBE), ou un mélange de tert-butyl éther avec un solvant aromatique fluoré, notamment le trifluorotoluène (BTF) et l'hexafluorobenzène (BHF), un solvant comprenant au moins un atome de silicium, notamment l'hexaméthyldisiloxane (HMDS) et le tétraméthylsilane (TMS), ou le méthyl-tétrahydrofurane (MeTHF), ou un mélange de ceux-ci.

Le premier système de solvants peut comprendre une teneur en tert-butyl éther, notamment le 2-éthoxy-2-méthylpropane, encore appelé éthyl-tert-butyl-éther (ETBE), ou un mélange de tert-butyl éther avec des solvants aromatiques fluorés, notamment le trifluorotoluène (BTF) et l'hexafluorobenzène (BHF), des solvants comprenant au moins un atome de silicium, notamment l'hexaméthyldisiloxane (HMDS) et le tétraméthylsilane (TMS) ou le méthyl-tétrahydrofurane (MeTHF), d'au moins 50 %, notamment au moins 75%, en particulier au moins 90%, plus particulièrement au moins 95%, encore plus particulièrement au moins 99% en volume par rapport au volume total du premier système de solvants.

Selon une variante, le premier système de solvants est constitué de tert-butyl éther, notamment le 2-éthoxy-2-méthylpropane, encore appelé éthyl-tert-butyl-éther (ETBE).

Selon une deuxième variante, le premier système de solvants est constitué de tert-butyl éther, notamment le 2-éthoxy-2-méthylpropane, encore appelé éthyl-tert-butyl-éther (ETBE) et d'un deuxième solvant, choisi parmi les solvants aromatiques fluorés, notamment le trifluorotoluène (BTF) et l'hexafluorobenzène (BHF), les solvants comprenant au moins un atome de silicium, notamment l'hexaméthyldisiloxane (HMDS) et le tétraméthylsilane (TMS) et le méthyl-tétrahydrofurane (MeTHF).

Selon une troisième variante, le premier système de solvants est constitué d'un ou plusieurs solvant(s) choisi parmi le HMDS, le HFB et le BTF et d'un ou plusieurs solvant(s) choisi parmi le le MTBE et l'ETBE.

Par ailleurs, la proportion de HMDS mise en oeuvre dans le premier système de solvants peut jouer un rôle important dans la consommation de solvants, d'eau de lavage et/ou de temps d'extraction. Il peut également faciliter la décantation et ainsi provoquer une formation d'émulsions moindre et/ou un déphasage plus rapide au cours des étapes d'extraction et/ou de lavage.

Selon une quatrième variante, le premier système de solvants est constitué d'ETBE.

Selon un mode de réalisation particulier, le premier système de solvants comprend une teneur en solvant(s) CMR, en particulier présent(s) sur la liste CMR UE1, UE2, et/ou US, inférieure ou égale à 10%, notamment inférieure ou égale à 5%, en particulier inférieure ou égale à 2%, tout particulièrement inférieure ou égale à 1%, encore plus particulièrement inférieure ou égale à 0,5%, voire inférieure ou égale à 0,1% en volume par rapport au volume total du premier système de solvants.

Encore plus particulièrement le premier système de solvants est dépourvu de solvants présents sur la liste CMR UE1, UE2 et/ou US.

Les solvants utilisés dans le premier système de solvants présentent une pureté d'au moins 90%, notamment d'au moins 95%, en particulier d'au moins 98%, tout particulièrement d'au moins 99%, voire d'au moins 99,5%.

En particulier, l'étape A) de transformation d'huile, de beurre ou de co-produit de l'industrie du raffinage des huiles végétales en solution hydro-alcoolique, notamment via une étape choisie parmi les saponifications et les estérifications, est effectuée dans un deuxième système de solvants comprenant une teneur en solvant choisi parmi les alcools de C2 à C4, et notamment l'éthanol, le n-propanol, l'isopropanol, le butanol, en particulier le n-butanol, le MeTHF et leurs mélanges d'au moins 50% en volume par rapport au volume total du deuxième système de solvants.

Le deuxième système de solvants peut comprendre une teneur en solvant choisi parmi les alcools de C2 à C4, et notamment l'éthanol, le n-propanol, l'iso-propanol, le butanol, en particulier le n-butanol, le MeTHF et leurs mélanges d'au moins 60 %, notamment au moins 75%, en particulier au moins 90%, plus particulièrement au moins 95%, encore plus particulièrement au moins 99% en volume par rapport au volume total du deuxième système de solvants.

En particulier, le deuxième système de solvants est constitué d'éthanol, de n-propanol, d'isopropanol, de butanol, de MeTHF ou d'un mélange de ceux-ci.

Le deuxième système de solvants peut comprendre une teneur en un solvant choisi parmi les alcools de C2 à C4, et notamment l'éthanol, le n-propanol, l'iso-propanol, le butanol, en particulier le n-butanol, et le MeTHF, d'au moins 50 %, notamment au moins 75%, en particulier au moins 90%, plus particulièrement au moins 95%, encore plus particulièrement au moins 99% en volume par rapport au volume total du deuxième système de solvants.

Selon un mode de réalisation particulier, le deuxième système de solvants comprend une teneur en solvant(s) présent(s) sur la liste CMR UE1, UE2 et/ou US, inférieure ou égale à 10%, notamment inférieure ou égale à 5%, en particulier inférieure ou égale à 2%, tout particulièrement inférieure ou égale à 1%, encore plus particulièrement inférieure ou égale à 0,5%, voire inférieure ou égale à 0,1% en volume par rapport au volume total du deuxième système de solvants.

Encore plus particulièrement le deuxième système de solvants est dépourvu de solvants présents sur la liste CMR UE1, UE2 et/ou US.

Les solvants utilisés dans le deuxième système de solvants présentent une pureté d'au moins 90%, notamment d'au moins 95%, en particulier d'au moins 98%, tout particulièrement d'au moins 99%, voire d'au moins 99,5%.

Par « solution hydroalcoolique diluée (SHD) », on entend au sens de la présente invention le milieu réactionnel de saponification dilué et comprenant notamment de l'eau et un ou plusieurs solvants très polaires, en particulier choisis parmi les alcools, par exemple en C2 à C4, et le MeTHF.

La solution hydroalcoolique diluée (SHD) extraite peut comprendre une teneur en eau supérieure ou égale à 50%, notamment supérieure ou égale à 60%, en particulier supérieure ou égale à 65%, tout particulièrement supérieure ou égale à 70%, voire supérieure ou égale à 72% en volume par rapport au volume de la solution hydroalcoolique.

La solution hydroalcoolique diluée (SHD) à extraire peut comprendre une teneur en eau inférieure ou égale à 95%, notamment inférieure ou égale à 90%, en particulier inférieure ou égale à 85%, tout particulièrement inférieure ou égale à 80%, voire inférieure ou égale à 75% en volume par rapport au volume de la solution hydroalcoolique.

Le rapport (volume/volume) SHD à extraire / système de solvants peut aller de 0,1 à 10, notamment de 0,25 à 5, en particulier de 0,5 à 2.

Le procédé selon l'invention peut permettre l'obtention d'une fraction insaponifiable sensiblement identique à la fraction insaponifiable obtenue par un procédé « classique » tel que décrit dans l'exemple 1 ci-dessous utilisant le 1,2-dichloroéthane ou le 1-chlorobutane.

Par « fraction insaponifiable sensiblement identique », on entend au sens de la présente invention une fraction insaponifiable qui présente un profil chromatographique et/ou une composition proche(s) d'une fraction de référence obtenue par une extraction au DCE ou au 1-chlorobutane, notamment l'extraction au DCE ou au 1-chlorobutane classique.

Par « profil chromatographique proche », on entend au sens de la présente invention un profil chromatographique caractérisé en ce que le dit profil chromatographique comporte toutes les familles de l'insaponifiable observées dans la fraction de référence obtenue par un procédé d'extraction classique au 1,2-dichloroéthane ou au 1-chlorobutane.

Par « composition proche », on entend au sens de la présente invention une composition caractérisée en ce que la teneur en composés des différentes familles de l'insaponifiable extrait est du même ordre de grandeur que celle observée dans la fraction de référence obtenue par un procédé d'extraction classique au 1,2-dichloroéthane ou au 1-chlorobutane, et plus particulièrement correspondant à la teneur suivante par rapport à la fraction de référence (% en masse par rapport à la masse de la fraction de référence) :

**Avocat**

| | |
|---|---|
| Composés furaniques | +/- 15 % |
| Alcools tri-hydroxylés | +/- 2 % |
| Hydrocarbures | +/- 0,5 % |
| Squalène | +/- 0,5 % |
| Stérols | +/- 1,5 % |
| Non Identifié | +/- 5 % |

**Soja**

| | |
|---|---|
| Hydrocarbures | +/- 0,5 % |
| Squalène | +/- 2 % |
| Tocophérols | +/- 8 % |
| Stérols | +/- 10 % |
| Non Identifié | +/- 3 % |

Dans le cas présent « +/- Y % » signifie que, dans le cas où la valeur de référence est X %, les teneurs peuvent aller de (X-Y) % à (X+Y) %. Dans le cas où X est 70 % et Y 15 %, la teneur peut aller de 55 % à 85 %.

En l'occurrence, la partie « Non Identifiée » peut ne pas être comprise dans la gamme spécifiée ci-dessus.

Par « extraction classique au 1,2-dichloroéthane ou au 1-chlorobutane », on entend au sens de la présente invention une extraction au 1,2 dichloroéthane ou au 1-chlorobutane suivant le procédé défini dans l'exemple 1, à partir de la même matière première que celle utilisée au cours de l'extraction d'un insaponifiable avec un autre système de solvants.

Par « fraction de référence », on entend au sens de la présente invention un insaponifiable extrait au 1,2 dichloroéthane ou au 1-chlorobutane, à partir de la même matière première que celle utilisée au cours de l'extraction d'un insaponifiable avec un autre système de solvants.

Par exemple dans le cas de l'avocat et du soja selon les protocoles décrits en exemple 1 dans le brevet EP 1 246 633 B1.

La présente invention a encore pour objet un procédé d'extraction d'une fraction insaponifiable, notamment partielle ou totale, contenue dans une huile végétale, une huile provenant d'un micro-organisme, un beurre végétal ou un co-produit de l'industrie du raffinage des huiles végétales, notamment d'avocat et/ou de soja, ou des huiles provenant de micro-organismes comprenant au moins
A) une étape de saponification par laquelle lesdites huiles, ledit beurre ou ledit co-produit de l'industrie du raffinage des huiles végétales ou des huiles provenant de micro-organimes est transformé en une solution hydro-alcoolique,
B) une étape d'extraction de la solution hydro-alcoolique par un premier système de solvants tel que défini ci-dessus.

Plus particulièrement, le procédé d'extraction de la fraction insaponifiable d'une huile de soja selon la présente invention est tel que l'on effectue une extraction liquide / liquide en mettant en contact la SHD avec un premier système de solvants, notamment à contre-courant, de la SHD au moyen d'un premier système de solvants tel que défini ci-dessus, en particulier constitué de BTF, le rapport (volume/volume) système de solvants / SHD allant de 0,1 à 10, notamment de 0,25 à 5, en particulier de 0,5 à 2.

Plus particulièrement, le procédé d'extraction de la fraction insaponifiable d'une huile d'avocat selon la présente invention est tel que l'on effectue une extraction liquide / liquide en mettant en contact la SHD avec un premier système de solvants, notamment une extraction à contre-courant de la SHD au moyen d'un système de solvants, en particulier constitué d'un mélange d'ETBE, le rapport (volume/volume) système de solvants / SHD allant de 0,1 à 10, notamment de 0,25 à 5, en particulier de 0,5 à 2.

L'huile végétale ou l'huile provenant de micro-organisme utilisée dans le présent procédé peut être choisie parmi l'huile de soja, de quinoa, de colza, de maïs, de tournesol, de sésame, de lupin, de coton, de noix de coco, d'olive, de palme, de germe de blé, de luzerne, d'avocat, de palmiste, d'arachide, de coprah, de lin, de ricin, de pépins de raisin, de pépins de courge, de pépins de cassis, de pépins de melon, de pépins de tomate, de pépins de citrouille, d'amande, de noisette, de noix, d'onagre, de bourrache, de carthame, de cameline, d'oeillette, de macro-algues, de micro-algues, comme Chorella, et/ou provenir de micro-organismes, notamment marin, d'eau douce ou terrestre, en particulier de levures, de moisissures, et plus particulièrement, de bactéries, et leurs mélanges.

Le beurre végétal peut être choisi parmi le beurre de cacao, d'illipé, de karité, et leurs mélanges.

La comparaison des teneurs en insaponifiables de différentes huiles végétales : soja, coton, noix de coco, olive et avocat, montre que l'huile d'avocat obtenue par extraction suivant divers procédés connus comprend un taux particulièrement important d'insaponifiable.

Typiquement, les teneurs de fraction insaponifiable obtenues s'échelonnent de 2 à 10% dans l'huile d'avocat, sont d'environ 0,5% dans l'huile de coco, d'environ 1% dans l'huile de soja et d'environ 1 % dans l'huile d'olive.

L'insaponifiable d'avocat peut être préparé par extraction à partir d'huile d'avocat.

Le procédé d'extraction de l'insaponifiable d'une huile d'avocat peut être effectué de la façon suivante.

Selon une méthode connue de l'homme du métier :
- soit on presse la pulpe fraîche en présence d'un tiers corps fibreux absorbant l'eau, tel que la parche de café, dans une presse à cage, puis on sépare l'émulsion d'huile et d'eau obtenue par décantation et/ou centrifugation;
- soit on broie la pulpe fraîche et on la met en contact avec un solvant organique adapté (par exemple un mélange méthanol-chloroforme) puis on récupère l'huile par évaporation du solvant. Plusieurs procédés ont été décrits dans l'art antérieur pour extraire la fraction insaponifiable d'une huile végétale.

On peut citer en particulier le procédé de préparation d'insaponifiable d'huile d'avocat tel que décrit et revendiqué dans le brevet FR 2 678 632 au nom des Laboratoires Pharmascience. Ce procédé permet d'obtenir un insaponifiable d'avocat riche en fraction furanique, encore appelée fraction H, en comparaison aux procédés classiques de préparation d'insaponifiable d'avocat.

Ainsi, l'insaponifiable d'huile d'avocat utilisé selon l'invention peut être obtenu à partir du fruit frais mais, de préférence, l'insaponifiable d'avocat est préparé à partir du fruit préalablement traité thermiquement, avant l'extraction de l'huile et la saponification, comme décrit dans le brevet FR 2 678 632.

Ce traitement thermique consiste en un séchage contrôlé du fruit, frais de préférence, pendant au moins quatre heures, avantageusement au moins 10 heures, de préférence entre environ 24 et environ 48 heures, à une température de préférence d'au moins environ 80°C et de préférence comprise entre environ 80 et environ 120°C.

On peut également citer le procédé de préparation d'insaponifiable d'huile de soja, obtenu à partir d'un concentrât d'insaponifiable d'huile de soja.

Ledit concentrât d'insaponifiable est préparé par distillation moléculaire selon un procédé tel que décrit pour l'huile de lupin dans la demande de brevet FR 2 762 512, mais adapté à l'huile de soja.

Dans ce procédé, l'huile de soja est distillée dans un distillateur moléculaire de type centrifuge ou à film raclé, à une température comprise entre environ 210 et 250°C et sous un vide poussé, compris entre 0,01 et 0,001 millimètres de mercure (soit 0,13 à 1,3 Pa).

Le distillat obtenu présente une teneur en insaponifiable comprise entre 5 et 40% en volume et constitue donc un concentrât d'insaponifiable d'huile de soja.

Le concentrât est ensuite saponifié par une base telle que la potasse ou la soude en milieu polaire, notamment alcoolique, de préférence de l'éthanol, du n-propanol, de l'iso-propanol, du butanol, en particulier le du n-butanol, du MeTHF, ou un mélange de ceux-ci, puis il est soumis à une ou plusieurs extractions par le premier système de solvants.

La solution d'extraction obtenue est de préférence ensuite centrifugée, filtrée puis lavée à l'eau pour éliminer les traces résiduelles d'alcalinité.

Le solvant d'extraction est évaporé soigneusement pour récupérer l'insaponifiable..

Enfin, avant sa saponification, l'huile peut être préalablement enrichie en insaponifiable en séparant une majorité des constituants de l'insaponifiable que l'on récupère dans un concentrât. Différentes méthodes peuvent être utilisées : cristallisation par le froid, extraction liquide / liquide, ou encore distillation moléculaire.

La concentration préalable de l'huile en insaponifiable permet de diminuer les volumes d'huile à saponifier.

La distillation moléculaire est particulièrement préférée, étant réalisée de préférence à une température comprise entre environ 180 et environ 230°C en maintenant une pression comprise entre 10⁻³ et 10⁻² mm Hg et de préférence de l'ordre de 10⁻³ mm Hg.

La concentration en insaponifiable du distillat peut atteindre 60 % en masse par rapport à la masse totale.

Tout particulièrement, la présente invention concerne un procédé tel que décrit ci-dessus dans lequel l'insaponifiable obtenu est choisi parmi un insaponifiable de soja, un insaponifiable d'avocat, notamment un insaponifiable d'avocat riche en fraction furanique et/ou un insaponifiable d'avocat riche en fraction stérolique, et encore plus particulièrement un mélange d'insaponifiables d'avocat et de soja (ASU).

Le procédé selon la présente invention permet d'extraire une fraction insaponifiable contenue dans une huile végétale, une huile provenant d'un micro-organisme ou un beurre végétal, il peut également permettre d'extraire une fraction insaponifiable à partir d'un co-produit de l'industrie du raffinage des huiles végétales ou des huiles provenant d'un micro-organisme, comme par exemple les échappées de désodorisation, encore appelées déodistillats, produites au cours du raffinage des huiles végétales ou des huiles provenant de micro-organismes.

Les acides gras et les glycérides partiels présents dans les déodistillats peuvent en effet être saponifiés ou estérifiés par un alcool léger, dans le but de séparer la fraction grasse de la fraction insaponifiable, soit par extraction liquide / liquide soit par distillation sous vide.

Enfin, la purification de l'insaponifiable ou des fractions actives séparées, le plus souvent les tocophérols (incluant la vitamine E) et les stérols, met en jeu notamment des étapes de cristallisation dans un solvant organique ou d'extraction liquide / liquide.

La présente invention a également pour objet un procédé d'extraction de la fraction insaponifiable dans un co-produit de l'industrie du raffinage d'une huile végétale ou d'une huile provenant d'un micro-organisme, tel que ce co-produit est un déodistillat d'une huile végétale ou d'une huile provenant d'un micro-organisme, ledit procédé comprenant au moins :
- une étape de saponification transformant le déodistillat en une solution hydro-alcoolique,
- une étape d'extraction à contre-courant de la solution hydro-alcoolique au moyen du premier système de solvants,
- une étape de cristallisation des stérols et/ou des alcools triterpéniques,
- une étape de séparation d'un composé actif, tel que les tocophérols, les tocotriénols, le squalène et les carotènes, ladite étape de séparation étant choisie dans le groupe formé par les extractions, en particulier au moyen du premier système de solvants, et les distillations.

Tout particulièrement, la cristallisation des stérols et/ou des alcools triterpéniques peut être effectuée dans le premier système de solvants.

La présente description concerne également une fraction insaponifiable, notamment partielle ou totale, dépourvue de solvants classés dans la liste CMR UE1, UE2 et/ou US, en particulier ladite fraction est obtenue par le procédé d'extraction selon la présente invention.

La présente description concerne encore l'utilisation de cette fraction pour la préparation d'une composition, notamment pharmaceutique, alimentaire et/ou cosmétique, ou encore d'un complément alimentaire.

La présente description concerne également une composition, notamment alimentaire, cosmétique ou pharmaceutique, ou encore un complément alimentaire, comprenant au moins une fraction insaponifiable d'au moins une huile végétale ou d'une huile provenant d'un micro-organisme, ladite fraction étant dépourvue de solvants classés dans la liste CMR UE1, UE2 et/ou US et/ou ladite fraction étant susceptible d'être obtenue, ou directement obtenue, par le procédé selon l'invention, et ladite composition comprenant éventuellement un excipient, en particulier cosmétiquement, alimentairement ou pharmaceutiquement acceptable.

Selon un mode de réalisation particulier, la présente description concerne une composition, notamment pharmaceutique, alimentaire ou cosmétique, ou encore un complément alimentaire, comprenant au moins un insaponifiable, en particulier un insaponifiable de soja, un insaponifiable d'avocat, tout particulièrement un insaponifiable d'avocat riche en fraction furanique et/ou un insaponifiable d'avocat riche en fraction stérolique, et encore plus particulièrement un mélange d'insaponifiables d'avocat et de soja (ASU) susceptible d'être obtenu ou directement obtenu par le procédé selon l'invention.

Les compositions pharmaceutiques peuvent être destinées à la prévention et/ou au traitement de troubles du tissu conjonctif, notamment de l'arthrose, des parodonthopathies et/ou du vieillissement cutané.

Les compositions alimentaires, ou compléments alimentaires peuvent être destinées à la prévention et/ou au traitement de troubles du tissu conjonctif, notamment de l'arthrose, des parodonthopathies, du vieillissement cutané et/ou et inflammation cutanée.

Les compositions cosmétiques peuvent être destinées à la prévention et/ou au traitement des troubles cutanées de l'épiderme du derme et/ou de l'hypoderme.

Par « dépourvu de solvants classés dans la liste CMR UE1, UE2 et/ou US », on entend au sens de la présente invention une teneur totale en solvants classés dans la liste CMR UE1, UE2 et/ou US inférieure à 10 ppm, notamment inférieure à 5 ppm, en particulier inférieure à 2 ppm, voire inférieure à 1 ppm.

La présente description vise encore un procédé de traitement cosmétique tel que l'on applique de façon topique la composition cosmétique selon l'invention et aussi l'utilisation d'un insaponifiable d'une huile végétale, d'une huile provenant d'un micro-organisme ou d'un beurre végétal obtenu selon la présente invention pour la fabrication d'un médicament, en particulier destiné à traiter ou à prévenir des troubles du tissu conjonctif, et notamment l'arthrose.

Bien entendu, les différentes caractéristiques exposées dans la présente description peuvent être combinées entre elles.

A titre d'exemples illustrant la présente invention, les expérimentations suivantes ont été effectuées.

### EXEMPLES

### Exemple 1 : Extraction d'insaponifiable d'avocat avec du DCE (référence 1)

La première étape consiste à saponifier un concentrât préparer par distillation moléculaire d'huile d'avocat.

A cet effet, dans un ballon de 100 ml équipé d'un réfrigérant sont successivement introduits une masse donnée de concentrât d'huile d'avocat (45,6 g) puis de l'éthanol (36,6 g), de la potasse à 50 % (5,2 g) et quelques grains de pierre ponce.

Le système est ensuite porté à reflux pendant 3h30 puis, après refroidissement, dilué avec de l'eau déminéralisée (60 ml).

Après saponification, une solution hydro-alcoolique est obtenue contenant l'insaponifiable (ou fraction d'insaponifiable) en solution. Cet insaponifiable est alors extrait par un premier système de solvants, en l'occurrence le DCE.

Plusieurs extractions successives (5 x 70 ml) sont réalisées ; les phases organiques ainsi collectées sont ensuite rassemblées et lavées à l'eau de ville (5 x 70 ml) jusqu'à neutralité (test à la phénolphtaléïne).

La phase solvant obtenue est alors séchée sur sulfate de sodium anhydre puis filtrée ; l'insaponifiable est ensuite récupéré par évaporation du solvant à l'évaporateur rotatif. L'insaponifiable ainsi extrait est pesé et stocké dans un pilulier sous atmosphère inerte.

La composition de l'insaponifiable extrait est ensuite mesurée par CPG (Chromatographie Phase Gazeuse). Les résultats sont présentés dans le tableau 1 ci-après.

### Exemple comparatif 2 : Extraction d'insaponifiable d'avocat avec du HMDS

L'extraction est effectuée selon le mode opératoire de l'Exemple 1 qui, après optimisation pour l'adapter au nouveau système de solvants, correspond à une étape d'extraction avec 4 x 70 ml de l'HMDS au lieu du DCE et une étape de lavage des phases organiques avec 3 x 70 ml d'eau. Les mesures sont effectuées comme dans l'Exemple 1 et les résultats sont présentés dans le tableau 1 ci-après.

### Exemple comparatif 3 : Extraction d'insaponifiable d'avocat avec un mélange HMDS-MeTHF (90/10 en volume)

L'extraction est effectuée selon le mode opératoire de l'Exemple 1 qui, après optimisation pour l'adapter au nouveau système de solvants, correspond à une étape d'extraction avec 4 x 70 ml d'un mélange HMDS-MeTHF (90/10 en volume) au lieu du DCE et une étape de lavage des phases organiques avec 2 x 70 ml d'eau. Les mesures sont effectuées comme dans l'Exemple 1 et les résultats sont présentés dans le tableau 1 ci-après.

### Exemple comparatif 4 : Extraction d'insaponifiable d'avocat avec un mélange HMDS-MeTHF (50/50 en volume)

L'extraction est effectuée selon le mode opératoire de l'Exemple 1 qui, après optimisation pour l'adapter au nouveau système de solvants, correspond à une étape d'extraction avec 6 x 70 ml d'un mélange HMDS-MeTHF (50/50 en volume) au lieu du DCE et une étape de lavage des phases organiques avec 8 x 70 ml d'eau. Les mesures sont effectuées comme dans l'Exemple 1 et les résultats sont présentés dans le tableau 1 ci-après.

**Tableau 1**

| Analyse - Profil chromatographique et composition de l'insaponifiable extrait selon les exemples 1 à 4 | | | | | |
|---|---|---|---|---|---|
| | | Ex.1 | Ex.2 | Ex. 3 | Ex. 4 |
| Composés spécifiques | Composés furaniques | 63,7 | 80,3 | 77,6 | 52,4 |
| | Alcools tri-hydroxylés | 8,6 | 0,4 | 1 | 14,0 |
| Hydrocarbures | | 1,2 | 1,4 | 1,4 | 1,1 |
| Squalène | | 1,1 | 1,4 | 1,3 | 1,0 |
| Tocophérols | | Traces | Traces | Traces | Traces |
| Stérols | | 3,5 | 1,2 | 2,4 | 3,4 |
| Autres | | 11,6 | 12,3 | 10,0 | 9,7 |
| **Rendement massique par rapport au concentrât** | | **39%** | **28%** | **29%** | **45%** |

Rendement massique par rapport au concentrât = 100 x (Masse d'insaponifiable extrait/ Masse de concentrât mis enjeu)

Les mélanges testés permettent d'aboutir à une extraction de l'insaponifiable. Tous les insaponifiables obtenus présentent un profil chromatographique proche de celui observé sur le produit de référence extrait au DCE.

Cependant leur composition met en avant l'influence des caractéristiques de polarité du premier système de solvants sur le potentiel de sélectivité vis à vis des constituants de l'insaponifiable extrait. Avec le mélange le plus polaire, les composés les plus polaires sont extraits en proportion plus importante.

Le HMDS utilisé seul permet une extraction sélective des composés apolaires.

Ainsi, un mélange HMDS/MeTHF permet d'extraire un insaponifiable présentant un profil chromatographique proche de celui de l'insaponifiable de référence. La composition de l'insaponifiable extrait est directement liée à la composition du système de solvants et plus particulièrement à sa polarité : plus le système de solvants est polaire, plus l'insaponifiable sera riche en composés polaires tels que les alcools tri-hydroxylés ; inversement, plus le système de solvants sera apolaire et plus l'extraction sera sélective et l'insaponifiable riche en composés apolaires tels les composés furaniques.

De plus, la composition du premier système de solvants, de par sa faible polarité, permet d'améliorer les étapes d'extraction et de lavage en limitant les quantités de solvants et/ou d'eau mises en oeuvre ainsi qu'en facilitant le déphasage.

### Exemple 5 : Extraction d'insaponifiable d'avocat avec de l'ETBE

L'extraction est effectuée selon le mode opératoire de l'Exemple 1 qui, après optimisation pour l'adapter au nouveau système de solvants, correspond à une étape d'extraction avec 4 x 60 ml d'ETBE au lieu du DCE et une étape de lavage des phases organiques avec 4 x 70 ml d'eau. Les mesures sont effectuées comme dans l'Exemple 1 et les résultats sont présentés dans le tableau 2 ci-après.

**Tableau 2**

| Analyse - Profil chromatographique et composition de l'insaponifiable extrait selon les exemples 1 et 5 | | | |
|---|---|---|---|
| | | Ex.1 | Ex. 5 |
| Composés spécifiques | Composés furaniques | 63,7 | 63,8 |
| | Alcools tri-hydroxylés | 8,6 | 7,1 |
| Hydrocarbures | | 1,2 | 1,2 |
| Squalène | | 1,1 | 1,1 |
| Tocophérols | | Traces | Traces |
| Stérols | | 3,5 | 3,0 |
| Autres | | 11,6 | 11,4 |
| **Rendement rapport au massique par concentrât** | | **39%** | **36%** |

Le premier système de solvants testé permet d'aboutir à une extraction d'insaponifiable avec un rendement massique voisin de celui obtenu lors d'une extraction classique au DCE. L'insaponifiable obtenu présente un profil chromatographique et une composition proches de ceux observés sur le produit de référence extrait au DCE.

De plus l'ETBE permet de diminuer le nombre d'extractions et donc la quantité de solvants ainsi que le nombre de lavages et donc la quantité d'eau à mettre en oeuvre pour extraire la fraction insaponifiable d'un concentrât d'avocat.

En conclusion, l'ETBE se présente comme une bonne alternative à l'utilisation du DCE en tant que premier système de solvants dans le procédé d'extraction d'insaponifiable d'avocat.

### Exemple comparatif 6 : Extraction d'insaponifiable d'avocat avec du 1-chlorobutane (référence 2)

L'extraction et le lavage sont effectués selon le mode opératoire de l'Exemple 1 avec du 1-chlorobutane au lieu du DCE. Les mesures sont effectuées comme dans l'Exemple 1 et les résultats sont présentés dans le tableau 3 ci-après.

### Exemple comparatif 7 : Extraction d'insaponifiable d'avocat avec du BTF

La saponification est réalisée selon le mode opératoire de l'Exemple 1 à ceci près que l'éthanol est remplacé par de l'iso-propanol (36,6 ml). L'extraction est effectuée selon le mode opératoire de l'Exemple 1 qui, après optimisation pour l'adapter au nouveau système de solvants, correspond à une étape d'extraction avec 4 x 35 ml de BTF au lieu du DCE et une étape de lavage des phases organiques avec 4 x 35 ml d'eau. Les mesures sont effectuées comme dans l'Exemple 1 et les résultats sont présentés dans le tableau 1 ci-après.

### Exemple comparatif 8 : Extraction d'insaponifiable d'avocat avec du HMDS

La saponification est réalisée selon le mode opératoire de l'Exemple 1 à ceci près que l'éthanol est remplacé par du MeTHF (36,6 ml). L'extraction est effectuée selon le mode opératoire de l'Exemple 1 qui, après optimisation pour l'adapter au nouveau système de solvants, correspond à une étape d'extraction avec 3 x 70 ml de HMDS au lieu du DCE et une étape de lavage des phases organiques avec 3 x 70 ml d'eau. Les mesures sont effectuées comme dans l'Exemple 1 et les résultats sont présentés dans le tableau 3 ci-après.

**Tableau 3**

| Analyse - Profil chromatographique et composition de l'insaponifiable extrait selon les exemples 6 à 8 | | | | |
|---|---|---|---|---|
| | | Ex.6 | Ex.7 | Ex. 8 |
| Composés spécifiques | Composés furaniques | 60,7 | 72,5 | 70,0 |
| | Alcools tri-hydroxylés | 5,5 | 3,1 | 2,8 |
| Hydrocarbures | | 1,1 | 1,4 | 1,2 |
| Squalène | | 1,1 | 1,3 | 1,2 |
| Tocophérols | | Traces | Traces | Traces |
| Stérols | | 3,1 | 3,1 | 2,4 |
| Autres | | 14,0 | 9,8 | 14,8 |

L'utilisation du MeTHF en deuxième système de solvants couplé à celle du HMDS en premier système de solvants permet d'aboutir à une extraction d'insaponifiable. L'insaponifiable obtenu présente un profil chromatographique et une composition proches de ceux observés sur le produit de référence extrait au 1-chlorobutane.

De même, l'utilisation de l'isopropanol en deuxième système de solvants couplé à celle du BTF en premier système de solvants permet d'aboutir à une extraction d'insaponifiable. L'insaponifiable obtenu présente un profil chromatographique et une composition proches de ceux observés sur le produit de référence extrait au 1-chlorobutane.

L'utilisation du MeTHF en deuxième système de solvants couplé à celle du HMDS en premier système de solvants d'une part et de l'isopropanol en deuxième système de solvants couplé à celle du BTF en premier système de solvants d'autre part permet de diminuer le nombre d'extractions et les quantités de solvants mises en oeuvre ainsi que le nombre de lavages et par la même les quantités d'eau à utiliser.

En conclusion, les couples MeTHF/HMDS d'une part et isopropanol/BTF d'autre part se présentent comme de bonnes alternatives à l'utilisation du couple éthanol/1-chlorobutane en permettant l'extraction d'insaponifiable de profils et compositions proches du produit de référence tout en diminuant les quantités de solvants et eau utilisées.

### Exemple 9 : Extraction d'insaponifiable de soja avec du DCE

La première étape consiste à saponifier un concentrât de soja. A cet effet, dans un ballon de 100 ml équipé d'un réfrigérant sont successivement introduits une masse donnée de concentrât d'huile de soja (10,0 g) puis, de l'éthanol (23,3 ml), de la potasse à 50% (1,7 ml) et quelques grains de pierre ponce.
Le système est ensuite porté à reflux pendant 3h00 puis dilué avec de l'eau déminéralisée (60 ml).

Après saponification, une solution hydro-alcoolique est obtenue contenant l'insaponifiable (ou fraction d'insaponifiable) en solution. Cet insaponifiable est alors extrait par un premier système de solvants, en l'occurrence le DCE.
Plusieurs extractions successives (5 x 43 ml) sont réalisées; les phases organiques ainsi collectées sont ensuite rassemblées et lavées à l'eau (7 x 43 ml) jusqu'à neutralité (test à la phénolphtaléïne).

La phase solvant obtenue est alors séchée sur sulfate de sodium anhydre puis filtrée; l'insaponifiable est ensuite récupéré par évaporation du solvant à l'évaporateur rotatif. L'insaponifiable ainsi extrait est pesé et stocké dans un pilulier sous atmosphère inerte.
La composition de l'insaponifiable extrait est ensuite mesurée par CPG (Chromatographie Phase Gazeuse). Les résultats sont présentés dans le tableau 4 ci-après.

### Exemple comparatif 10 : Extraction d'insaponifiable de soja avec du BTF

La saponification est réalisée selon le mode opératoire de l'Exemple 9 à ceci près que le volume d'éthanol est augmenté (50,0 ml). L'extraction est effectuée selon le mode opératoire de l'Exemple 9 qui, après optimisation pour l'adapter au nouveau système de solvants, correspond à une étape d'extraction avec 7 x 35 ml de BTF au lieu du DCE et une étape de lavage des phases organiques avec 10 x 35 ml d'eau. Les mesures sont effectuées comme dans l'Exemple 9 et les résultats sont présentés dans le tableau 4 ci-après.

### Exemple comparatif 11 : Extraction d'insaponifiable de soja avec un mélange de HMDS-MeTHF (50/50 en volume)

L'extraction est effectuée selon le mode opératoire de l'Exemple 9 qui, après optimisation pour l'adapter au nouveau système de solvants, correspond à une étape d'extraction avec 5 x 43 ml d'un mélange de HMDS-MeTHF (50/50 en volume) au lieu du DCE et une étape de lavage des phases organiques avec 4 x 43 ml d'eau. Les mesures sont effectuées comme dans l'Exemple 9 et les résultats sont présentés dans le tableau 4 ci-après.

**Tableau 4**

| Analyse - Profil chromatographique et composition de l'insaponifiable extrait selon les exemples 9 à 11 | | | |
|---|---|---|---|
| | Ex.9 | Ex.10 | Ex. 11 |
| Hydrocarbures | 0,1 | 0,2 | 0,1 |
| Squalène | 4,4 | 4,5 | 3,9 |
| Tocophérols | 25,8 | 23,4 | 19,8 |
| Stérols | 44,3 | 43,7 | 41,0 |
| Autres | 6,0 | 6,1 | 4,6 |
| Rendement **massique par rapport au concentrât** | **45%** | **38%** | **49%** |

Les premiers systèmes de solvants testés permettent d'aboutir à une extraction d'insaponifiable. L'insaponifiable obtenu présente un profil chromatographique et une composition proches de ceux observés sur le produit de référence extrait au DCE.

En conclusion, le BTF et un mélange HMDS/MeTHF à 50% en volume se présentent comme de bonnes alternatives à l'utilisation du DCE.

### Exemple comparatif 12 : Extraction d'insaponifiable de colza avec du BTF

La saponification est réalisée selon le mode opératoire de l'Exemple 10 à ceci près que le concentrât considéré est un concentrât de colza. L'extraction est effectuée selon le mode opératoire de l'Exemple 10. Les mesures sont effectuées comme dans l'Exemple 10 et les résultats sont présentés dans les tableaux 5 ci-après.

### Exemple comparatif 13 : Extraction d'insaponifiable de maïs avec du BTF

La saponification est réalisée selon le mode opératoire de l'Exemple 10 à ceci près que le concentrât considéré est un concentrât de maïs. L'extraction est effectuée selon le mode opératoire de l'Exemple 10. Les mesures sont effectuées comme dans l'Exemple 10 et les résultats sont présentés dans les tableaux 5 ci-après.

### Exemple comparatif 14 : Extraction d'insaponifiable de tournesol avec du BTF

La saponification est réalisée selon le mode opératoire de l'Exemple 10 à ceci près que le concentrât considéré est un concentrât de tournesol. L'extraction est effectuée selon le mode opératoire de l'Exemple 10. Les mesures sont effectuées comme dans l'Exemple 10 et les résultats sont présentés dans les tableaux 5 ci-après.

**Tableau 5**

| Analyse - Profil chromatographique et composition de l'insaponifiable extrait selon les exemples 10 et 12 à 14 | | | | |
|---|---|---|---|---|
| | Ex.10 | Ex.12 | Ex. 13 | Ex. 14 |
| Tocophérols (%) | 23,4 | 6,4 | 7,9 | 9,7 |
| Stérols (%) | 43,7 | 63,1 | 55,3 | 58,7 |
| Rendement massique par rapport au concentrat | **38%** | **7,2%** | **9,0%** | **4,0%** |
| Rendement massique par rapport à l'insaponifiable | **79%** | **76%** | **83%** | **78%** |

Le BTF appliqué à différentes matières végétales donne des résultats équivalents en termes de rendement massique par rapport à l'insaponifiable.

### Exemple 15 : Extraction d'insaponifiable de sésame avec de l'ETBE

La saponification est réalisée selon le mode opératoire de l'Exemple 5 à ceci près que le concentrat considéré est un concentrat de sésame. L'extraction est effectuée selon le mode opératoire de l'Exemple 5. Les mesures sont effectuées comme dans l'Exemple 5 et les résultats sont présentés dans les tableaux 6 ci-après.

### Exemple comparatif 16 : Extraction d'insaponifiable de sésame avec un mélange HMDS-MeTHF (50/50 en volume)

La saponification est réalisée selon le mode opératoire de l'Exemple 4 à ceci près que le concentrât considéré est un concentrât de sésame. L'extraction est effectuée selon le mode opératoire de l'Exemple 4. Les mesures sont effectuées comme dans l'Exemple 4 et les résultats sont présentés dans le tableaux 6 ci-après.

**Tableau 6 Analyse - Profil chromatographique et composition de l'insaponifiable extrait selon les exemples 15 et 16**

| | Ex.15 | Ex.16 |
|---|---|---|
| Stérols (%) | 17,4 | 20,0 |
| Sésamine (%) | 28,0 | 31,4 |
| Sésamoline (%) | 13,3 | 15,7 |
| Rendement massique par rapport au concentrât | **12%** | **15%** |
| Rendement massique par rapport à l'insaponifiable | **83%** | **98%** |

L'ETBE et le mélange HMDS-MeTHF (50/50 en volume) sont tout deux de bon solvants d'extraction pour l'insaponifiable de sésame. Le mélange HMDS-MeTHF présente une excellente capacité extractive avec un rendement d'extraction de l'insaponifiable de 98% massique.

### Exemple comparatif 17 : Extraction d'insaponifiable de palme avec du BTF

La saponification est réalisée selon le mode opératoire de l'Exemple 10 à ceci près que le concentrât considéré est un concentrât de palme. L'extraction est effectuée selon le mode opératoire de l'Exemple 10. Les mesures sont effectuées comme dans l'Exemple 10 et les résultats sont présentés dans les tableaux 7 ci-après.

### Exemple comparatif 18 : Extraction d'insaponifiable de palme avec un mélange HMDS-MeTHF (50/50 en volume)

La saponification est réalisée selon le mode opératoire de l'Exemple 11 à ceci près que le concentrat considéré est un concentrat de palme. L'extraction est effectuée selon le mode opératoire de l'Exemple 11. Les mesures sont effectuées comme dans l'Exemple 11 et les résultats sont présentés dans le tableau 7 ci-après.

**Tableau 7**

| Analyse - Profil chromatographique et composition de l'insaponifiable extrait selon les exemples 17 et 18 | | |
|---|---|---|
| | Ex.17 | Ex.18 |
| Stérols (%) | 23,0 | 25,0 |
| Tocophérols (%) | 3,7 | 3,2 |
| Tocotrienols (%) | 11,4 | 8,9 |
| Carotènes | 0,6 | 0,5 |
| Rendement massique par rapport au concentrât | **2%** | **3%** |
| Rendement massique par rapport à l'insaponifiable | **75%** | **99%** |

Le BTF et le mélange HMDS-MeTHF (50/50 en volume) sont tout deux de bon solvants d'extraction pour l'insaponifiable de palme. Le mélange HMDS-MeTHF présente une excellente capacité extractive avec un rendement d'extraction de l'insaponifiable de 99% massique.

## Revendications

1. Procédé d'extraction d'une fraction insaponifiable contenue dans une huile végétale, une huile provenant d'un micro-organisme ou un beurre végétal, ou dans un co-produit de l'industrie du raffinage des huiles végétales ou des huiles provenant de micro-organismes, tel que les échappées de désodorisation, comprenant au moins :
A) une étape de transformation par saponification ou estérification desdites huiles, dudit beurre ou dudit co-produit de l'industrie du raffinage des huiles végétales ou des huiles provenant de micro-organismes en solution hydro-alcoolique, B) une étape d'extraction de la solution hydro-alcoolique dans laquelle la fraction grasse est séparée de la fraction insaponifiable par une extraction liquide / liquide,
ledit procédé étant **caractérisé en ce que** l'extraction liquide / liquide de l'étape B est effectuée en utilisant un premier système de solvants comprenant une teneur en tert-butyl éthers, notamment le 2-éthoxy-2-méthylpropane, encore appelé éthyl-tert-butyl-éther (ETBE), ou un mélange de tert-butyl éthers avec des solvants aromatiques fluorés, notamment le trifluorotoluène (BTF) et l'hexafluorobenzène (BHF), des solvants comprenant au moins un atome de silicium, notamment l'hexaméthyldisiloxane (HMDS) et le tétraméthylsilane (TMS) ou le méthyl-tétrahydrofurane (MeTHF), et leurs mélanges, d'au moins 50% en volume par rapport au volume total du système de solvants.

2. Procédé selon la revendication 1, **caractérisé en ce qu'**il comprend une étape C de purification de l'insaponifiable choisie dans le groupe constitué par les cristallisations et les extractions liquide / liquide.

3. Procédé selon la revendication 2, **caractérisé en ce que** les cristallisations de l'étape C ou les extractions liquide / liquide de l'étape C sont effectuées en utilisant un premier système de solvants comprenant une teneur en tert-butyl éthers, notamment le 2-éthoxy-2-méthylpropane, encore appelé éthyl-tert-butyl-éther (ETBE), ou un mélange de tert-butyl éthers avec des solvants aromatiques fluorés, notamment le trifluorotoluène (BTF) et l'hexafluorobenzène (BHF), des solvants comprenant au moins un atome de silicium, notamment l'hexaméthyldisiloxane (HMDS) et le tétraméthylsilane (TMS) ou le méthyl-tétrahydrofurane (MeTHF), et leurs mélanges, d'au moins 50% en volume par rapport au volume total du système de solvants.

4. Procédé selon les revendications 1 à 3, **caractérisé en ce que** le premier système de solvants comprend une teneur en tert-butyl éthers, notamment le 2-éthoxy-2-méthylpropane, encore appelé éthyl-tert-butyl-éther (ETBE), ou un mélange de tert-butyl éthers avec des solvants aromatiques fluorés, notamment le trifluorotoluène (BTF) et l'hexafluorobenzène (BHF), des solvants comprenant au moins un atome de silicium, notamment l'hexaméthyldisiloxane (HMDS) et le tétraméthylsilane (TMS), ou le méthyl-tétrahydrofurane (MeTHF), et leurs mélanges, d'au moins 60 %, notamment au moins 75%, en particulier au moins 90%, plus particulièrement au moins 95%, encore plus particulièrement au moins 99% en volume, par rapport au volume total du premier système de solvants.

5. Procédé selon l'une quelconque des revendications 1 à 4, **caractérisé en ce que** le premier système de solvants est constitué de tert-butyl éther, notamment le 2-éthoxy-2-méthylpropane, encore appelé éthyl-tert-butyl-éther (ETBE), ou un mélange de tert-butyl éther avec un solvant aromatique fluoré, notamment le trifluorotoluène (BTF) et l'hexafluorobenzène (BHF), un solvant comprenant au moins un atome de silicium, notamment l'hexaméthyldisiloxane (HMDS) et le tétraméthylsilane (TMS), le méthyl-tétrahydrofurane (MeTHF), ou un mélange de ceux-ci.

6. Procédé selon l'une quelconque des revendications 1 à 5, **caractérisé en ce que** l'étape A) de transformation par saponification ou estérification d'huile ou de beurre en solution hydro-alcoolique, est effectuée dans un deuxième système de solvants comprenant une teneur en solvant choisi parmi les alcools de C2 à C4, et notamment l'éthanol, le n-propanol, l'isopropanol, le butanol, en particulier le n-butanol, le MeTHF, et leurs mélanges d'au moins 50% en volume par rapport au volume total du système de solvants.

7. Procédé selon la revendication 6, **caractérisé en ce que** le deuxième système de solvants comprend une teneur en solvant choisi parmi les alcools de C2 à C4, et notamment l'éthanol, le n-propanol, l'iso-propanol, le butanol, en particulier le n-butanol, le MeTHF, et leurs mélanges d'au moins 60 %, notamment au moins 75%, en particulier au moins 90%, plus particulièrement au moins 95%, encore plus particulièrement au moins 99% en volume par apport au volume total du deuxième système de solvants.

8. Procédé selon la revendication 6 ou 7, **caractérisé en ce que** le deuxième système de solvants est constitué d'alcools de C2 à C4, et notamment d'éthanol, de n-propanol, d'iso-propanol, de butanol, en particulier le n-butanol, de MeTHF, ou d'un mélange de ceux-ci.

9. Procédé selon l'une quelconque des revendications 6 à 8, **caractérisé en ce que** le deuxième système de solvants comprend une teneur en un solvant choisi parmi les alcools de C2 à C4, et notamment l'éthanol, le n-propanol, l'iso-propanol, le butanol, en particulier le n-butanol, et le MeTHF, d'au moins 50 %, notamment au moins 75%, en particulier au moins 90%, plus particulièrement au moins 95%, encore plus particulièrement au moins 99% en volume par apport au volume total du deuxième système de solvants.

10. Procédé selon l'une quelconque des revendications 1 à 9, **caractérisé en ce que** l'huile végétale, l'huile provenant d'un micro-organisme ou le beurre végétal est choisi parmi l'huile de soja, de quinoa, de colza, de maïs, de tournesol, de sésame, de lupin, de coton, de noix de coco, d'olive, de palme, de germe de blé, de luzerne, d'avocat, de palmiste, d'arachide, de coprah, de lin, de ricin, de pépins de raisin, de pépins de courge, de pépins de cassis, de pépins de melon, de pépins de tomate, de pépins de citrouille, d'amande, de noisette, de noix, d'onagre, de bourrache, de carthame, de cameline, d'oeillette, de macro-algues, de micro-algues, comme Chorella, et/ou provenir de micro-organismes, notamment marin, d'eau douce ou terrestre, en particulier de levures, de moisissures, et plus particulièrement, de bactéries, et leurs mélanges, et le beurre de cacao, d'illipé, de karité, et leurs mélanges.

11. Procédé selon l'une quelconque des revendications 1 à 10, **caractérisé en ce que** le premier système de solvants est constitué par un tert-butyl éther, notamment le 2-éthoxy-2-méthylpropane, encore appelé éthyl-tert-butyl-éther (ETBE).

12. Procédé selon l'une quelconque des revendications 1 à 10 **caractérisé en ce que** le premier système de solvants est constitué d'un premier solvant choisi parmi les solvants aromatiques fluorés, notamment le trifluorotoluène (BTF) et l'hexafluorobenzène (BHF), les solvants comprenant au moins un atome de silicium, notamment l'hexaméthyldisiloxane (HMDS) et le tétraméthylsilane (TMS) ou le méthyl-tétrahydrofurane (MeTHF), et d'un deuxième solvant, différent du premier, choisi parmi les tert-butyl éthers, notamment le 2-éthoxy-2-méthylpropane, encore appelé éthyl-tert-butyl-éther (ETBE), et le 2-méthoxy-2-méthylpropane, encore appelé méthyl-tert-butyl éther (MTBE).

13. Procédé selon l'une quelconque des revendications 1 à 10, **caractérisé en ce que** le premier système de solvants est constitué d'un ou plusieurs solvant(s) choisi parmi HMDS, le HFB et le BTF et d'un ou plusieurs solvant(s) choisi parmi le MTBE et l'ETBE.

14. Procédé selon l'une quelconque des revendications 1 à 10, **caractérisé en ce que** le premier système de solvants est constitué d'ETBE.

15. Procédé selon l'une quelconque des revendications 1 à 10, **caractérisé en ce que** l'insaponifiable obtenu est choisi parmi un insaponifiable de soja, un insaponifiable d'avocat, notamment un insaponifiable d'avocat riche en fraction furanique et/ou un insaponifiable d'avocat riche en fraction stérolique, et plus particulièrement un mélange d'insaponifiables d'avocat et de soja (ASU).

## Patentansprüche

1. Verfahren zur Extraktion einer unverseifbaren Fraktion, die in einem Pflanzenöl, einem Öl, das von einem Mikroorganismus abgeleitet, oder einer Pflanzenbutter oder einem Nebenprodukt der Industrie der Raffination von Pflanzenölen oder von Ölen, die von Mikroorganismen abgeleitet sind, wie Desodorierungsausflüsse, enthalten ist, wobei das Verfahren mindestens Folgendes umfasst:
A) einen Schritt der Umwandlung durch Verseifung oder Veresterung der besagten Öle, der besagten Butter oder des besagten Nebenprodukts der Industrie der Raffination von Pflanzenölen oder von Ölen, die von Mikroorganismen abgeleitet sind, in einer wässrigalkoholischen Lösung, B) einen Schritt der Extraktion der wässrig-alkoholischen Lösung, in dem die Fettfraktion von der unverseifbaren Fraktion durch eine Flüssig-Flüssig-Extraktion getrennt wird,
wobei das besagte Verfahren **dadurch gekennzeichnet ist, dass** die Flüssig-Flüssig-Extraktion des Schritts B durchgeführt wird, indem ein erstes Lösungsmittelsystem verwendet wird, das einen Gehalt an tert.-Butylethern, insbesondere 2-Ethoxy-2-methylpropan, das auch als Ethyl-tert.-butylether (ETBE) bezeichnet wird, oder eines Gemischs von tert.-Butylethern mit fluorierten aromatischen Lösungsmitteln, insbesondere Trifluortoluol (BTF) und Hexafluorbenzol (BHF), Lösungsmitteln, Lösungsmitteln die mindestens ein Siliciumatom umfassen, insbesondere Hexamethyldisiloxan (HMDS) und Tetramethylsilan (TMS) oder Methyltetrahydrofuran (MeTHF), und deren Gemischen, von mindestens 50 Vol.-%, bezogen auf das Gesamtvolumen des Lösungsmittelsystems, umfasst.

2. Verfahren nach Anspruch 1, **dadurch gekennzeichnet, dass** es einen Schritt C der Aufreinigung des Unverseifbaren umfasst, der aus der Gruppe bestehend aus Kristallisationen und Flüssig-Flüssig-Extraktionen ausgewählt ist.

3. Verfahren nach Anspruch 2, **dadurch gekennzeichnet, dass** die Kristallisationen des Schritts C oder die Flüssig-Flüssig-Extraktionen des Schritts C durchgeführt werden, indem ein erstes Lösungsmittelsystem verwendet wird, das einen Gehalt an tert.-Butylethern, insbesondere 2-Ethoxy-2-methylpropan, das auch als Ethyl-tert.-butylether (ETBE) bezeichnet wird, oder eines Gemischs von tert.-Butylethern mit fluorierten aromatischen Lösungsmitteln, insbesondere Trifluortoluol (BTF) und Hexafluorbenzol (BHF), Lösungsmitteln, die mindestens ein Siliciumatom umfassen, insbesondere Hexamethyldisiloxan (HMDS) und Tetramethylsilan (TMS), oder Methyltetrahydrofuran (MeTHF) und deren Gemischen, von mindestens 50 Vol.-%, bezogen auf das Gesamtvolumen des Lösungsmittelsystems, umfasst.

4. Verfahren nach einem der Ansprüche 1 bis 3, **dadurch gekennzeichnet, dass** das erste Lösungsmittelsystem einen Gehalt an tert.-Butylethern, insbesondere 2-Ethoxy-2-methylpropan, das auch als Ethyl-tert.-butylether (ETBE) bezeichnet wird, oder eines Gemischs von tert.-Butylethern mit fluorierten aromatischen Lösungsmitteln, insbesondere Trifluortoluol (BTF) und Hexafluorbenzol (BHF), Lösungsmitteln, die mindestens ein Siliciumatom umfassen, insbesondere Hexamethyldisiloxan (HMDS) und Tetramethylsilan (TMS), oder Methyltetrahydrofuran (MeTHF) und deren Gemischen, von mindestens 60 Vol.-%, insbesondere mindestens 75 Vol.-%, besonders mindestens 90 Vol.-%, ganz besonders mindestens 95 Vol.-%, noch mehr besonders mindestens 99 Vol.-%, bezogen auf das Gesamtvolumen des ersten Lösungsmittelsystems, umfasst.

5. Verfahren nach einem der Ansprüche 1 bis 4, **dadurch gekennzeichnet, dass** das erste Lösungsmittelsystem aus tert.-Butylether, insbesondere 2-Ethoxy-2-methylpropan, das auch als Ethyl-tert.-butylether (ETBE) bezeichnet wird, oder einem Gemisch von tert.-Butylether mit einem fluorierten aromatischen Lösungsmittel, insbesondere Trifluortoluol (BTF) und Hexafluorbenzol (BHF), einem Lösungsmittel, das mindestens ein Siliciumatom umfasst, insbesondere Hexamethyldisiloxan (HMDS) und Tetramethylsilan (TMS), Methyltetrahydrofuran (MeTHF) oder einem Gemisch dieser, besteht.

6. Verfahren nach einem der Ansprüche 1 bis 5, **dadurch gekennzeichnet, dass** der Schritt A) der Umwandlung durch Verseifung oder Veresterung eines Öls oder einer Butter in wässrig-alkoholischer Lösung in einem zweiten Lösungsmittelsystem durchgeführt wird, das einen Gehalt an einem Lösungsmittel, das aus C2-bis C4-Alkoholen und insbesondere Ethanol, n-Propanol, Isopropanol, Butanol, insbesondere n-Butanol, MeTHF und deren Gemischen ausgewählt ist, von mindestens 50 Vol.-%, bezogen auf das Gesamtvolumen des Lösungsmittelsystems, umfasst.

7. Verfahren nach Anspruch 6, **dadurch gekennzeichnet, dass** das zweite Lösungsmittelsystem einen Gehalt an einem Lösungsmittel, das aus C2- bis C4-Alkoholen und insbesondere Ethanol, n-Propanol, Isopropanol, Butanol, insbesondere n-Butanol, MeTHF und deren Gemischen ausgewählt ist, von mindestens 60 Vol.-%, insbesondere mindestens 75 Vol.-%, besonders mindestens 90 Vol.-%, ganz besonders mindestens 95 Vol.-%, noch mehr besonders mindestens 99 Vol.-%, bezogen auf das Gesamtvolumen des zweiten Lösungsmittelsystems, umfasst.

8. Verfahren nach Anspruch 6 oder 7, **dadurch gekennzeichnet, dass** das zweite Lösungsmittelsystem aus C2- bis C4-Alkoholen und insbesondere Ethanol, n-Propanol, Isopropanol, Butanol, insbesondere n-Butanol, MeTHF oder einem Gemisch dieser, besteht.

9. Verfahren nach einem der Ansprüche 6 bis 8, **dadurch gekennzeichnet, dass** das zweite Lösungsmittelsystem einen Gehalt an einem Lösungsmittel, das aus C2- bis C4-Alkoholen und insbesondere Ethanol, n-Propanol, Isopropanol, Butanol, insbesondere n-Butanol, und MeTHF ausgewählt ist, von mindestens 50 Vol.-%, insbesondere mindestens 75 Vol.-%, besonders mindestens 90 Vol.-%, ganz besonders mindestens 95 Vol.-%, noch mehr besonders mindestens 99 Vol.-%, bezogen auf das Gesamtvolumen des zweiten Lösungsmittelsystems, umfasst.

10. Verfahren nach einem der Ansprüche 1 bis 9, **dadurch gekennzeichnet, dass** das Pflanzenöl, das Öl, das von einem Mikroorganismus abgeleitet ist, oder die Pflanzenbutter aus Sojaöl, Quinoaöl, Rapsöl, Maisöl, Sonnenblumenöl, Sesamöl, Lupinenöl, Baumwollsaatöl, Kokosöl, Olivenöl, Palmöl, Weizenkeimöl, Luzernenöl, Avocadoöl, Palmkernöl, Erdnussöl, Kopraöl, Leinöl, Rizinusöl, Traubenkernöl, Kürbiskernöl, Johannisbeerkernöl, Melonenkernöl, Tomatensamenöl, Gartenkürbiskernöl, Mandelöl, Haselnussöl, Walnussöl, Nachtkerzenöl, Borretschöl, Färberdistelöl, Dotterblumenöl, Schlafmohnöl, Makroalgenöl, Mikroalgenöl, wie Chorella, und/oder Öl, das von Mikroorganismen abgeleitet ist, insbesondere aus dem Meer, Süßwasser oder Irdischer, insbesondere Hefen, Schimmelpilzen und ganz besonders Bakterien, und deren Gemischen, und Kakaobutter, Illipe-Butter, Sheabutter und deren Gemischen ausgewählt sind.

11. Verfahren nach einem der Ansprüche 1 bis 10, **dadurch gekennzeichnet, dass** das erste Lösungsmittelsystem aus einem tert.-Butylether, insbesondere 2-Ethoxy-2-methylpropan, das auch als Ethyl-tert.-butylether (ETBE) bezeichnet wird, besteht.

12. Verfahren nach einem der Ansprüche 1 bis 10, **dadurch gekennzeichnet, dass** das erste Lösungsmittelsystem aus einem ersten Lösungsmittel, das aus fluorierten aromatischen Lösungsmitteln, insbesondere Trifluortoluol (BTF) und Hexafluorbenzol (BHF), Lösungsmitteln, die mindestens ein Siliciumatom umfassen, insbesondere Hexamethyldisiloxan (HMDS) und Tetramethylsilan (TMS), oder Methyltetrahydrofuran (MeTHF) und deren Gemischen ausgewählt ist, und einem zweiten Lösungsmittel besteht, das sich von dem ersten unterscheidet und aus tert.-Butylethern, insbesondere 2-Ethoxy-2-methylpropan, das auch als Ethyl-tert.-butylether (ETBE) bezeichnet wird, und 2-Methoxy-2-methylpropan, das auch als Methyl-tert.-butylether (MTBE) bezeichnet wird, ausgewählt ist.

13. Verfahren nach einem der Ansprüche 1 bis 10, **dadurch gekennzeichnet, dass** das erste Lösungsmittelsystem aus einem oder mehreren Lösungsmitteln, das bzw. die aus HMDS, HFB und BHF und BTF ausgewählt ist bzw. sind, und einem oder mehreren Lösungsmitteln, das bzw. die aus MTBE und ETBE ausgewählt ist bzw. sind, besteht.

14. Verfahren nach einem der Ansprüche 1 bis 10, **dadurch gekennzeichnet, dass** das erste Lösungsmittelsystem aus ETBE besteht.

15. Verfahren nach einem der Ansprüche 1 bis 10, **dadurch gekennzeichnet, dass** das erhaltene Unverseifbare aus einem Unverseifbaren von Soja, einem Unverseifbaren von Avocado, insbesondere einem Unverseifbaren von Avocado, das reich an einer Furanfraktion ist, und/oder einem Unverseifbaren von Avocado, das reich an einer Sterolfraktion ist, und ganz besonders einem Gemisch von Unverseifbaren von Avocado und Soja (ASU) ausgewählt ist.

## Claims

1. Method for extracting an unsaponifiable fraction contained in a vegetable oil, an oil from a microorganism or a vegetable butter, or in a by-product of the industry involved in the refining of vegetable oils or oils from microorganisms, such as deodorization discharge, comprising at least:
A) a step of transformation by saponification or esterification of said oils, said butter or said by-product of the industry involved in the refining of vegetable oils or oils from microorganisms to an aqueous-alcoholic solution,
B) a step of extraction of the aqueous-alcoholic solution in which the fat fraction is separated from the unsaponifiable fraction by liquid-liquid extraction,
wherein said method is **characterized in that** liquid-liquid extraction of step B is carried out with a first solvent system comprising a content of tert-butyl ethers, notably 2-ethoxy-2-methylpropane, also called ethyl-tert-butyl-ether (ETBE), or a mixture of *tert-*butyl ethers with fluorinated aromatic solvents, notably trifluorotoluene (BTF) and hexafluorobenzene (BHF), solvents comprising at least one silicon atom, notably hexamethyldisiloxane (HMDS) and tetramethylsilane (TMS) or methyl-tetrahydrofuran (MeTHF), and mixtures thereof of at least 50% by volume in relation to the total volume of the solvent system.

2. Method according to claim 1, wherein it comprises a step C of purification of the unsaponifiable selected from the group comprising crystallizations and/or liquid-liquid extractions.

3. Method according to claim 2, wherein the crystallizations of step C or the liquid-liquid extractions of step C are carried out using a first solvent system comprising a content of tert-butyl ethers, notably 2-ethoxy-2-methylpropane, also called ethyl-tert-butyl-ether (ETBE), or a mixture of *tert-*butyl ethers with fluorinated aromatic solvents, notably trifluorotoluene (BTF) and hexafluorobenzene (BHF), solvents comprising at least one silicon atom, notably hexamethyldisiloxane (HMDS) and tetramethylsilane (TMS) or methyl-tetrahydrofuran (MeTHF), and mixtures thereof, of at least 50% by volume in relation to the total volume of the solvent system.

4. Method according to claims 1 to 3, wherein the first solvent system comprises a content of tert-butyl ethers, notably 2-ethoxy-2-methylpropane, also called ethyl-tert-butyl-ether (ETBE), or a mixture of *tert-*butyl ethers with fluorinated aromatic solvents, notably trifluorotoluene (BTF) and hexafluorobenzene (BHF), solvents comprising at least one silicon atom, notably hexamethyldisiloxane (HMDS) and tetramethylsilane (TMS) or methyl-tetrahydrofuran (MeTHF), and mixtures thereof, of at least 60%, notably at least 75%, in particular at least 90%, more particularly at least 95%, even more particularly at least 99% by volume in relation to the total volume of the first solvent system.

5. Method according to any one of claims 1 to 4, wherein the first solvent system consists of tert-butyl ether, notably 2-ethoxy-2-methylpropane, also called ethyl-*tert*-butyl-ether (ETBE), or a mixture of *tert-*butyl ethers with fluorinated aromatic solvents, notably trifluorotoluene (BTF) and hexafluorobenzene (BHF), solvents comprising at least one silicon atom, notably hexamethyldisiloxane (HMDS) and tetramethylsilane (TMS), methyl-tetrahydrofuran (MeTHF), or mixtures thereof.

6. Method according to any one of claims 1 to 5, wherein the step A) of transformation by saponification or esterification of oil or butter to an aqueous-alcoholic solution, is carried out in a second solvent system comprising a content of solvents selected from C₂-C₄ alcohols, and notably ethanol, n-propanol, isopropanol, butanol, in particular n-butanol, MeTHF and mixtures thereof of at least 50% by volume in relation to the total volume of the solvent system.

7. Method according to claim 6, wherein the second solvent system comprises a content of solvents selected from C₂-C₄ alcohols, and notably ethanol, n-propanol, isopropanol, butanol, in particular n-butanol, MeTHF and mixtures thereof of at least 60%, notably at least 75%, in particular at least 90%, more particularly at least 95%, even more particularly at least 99% by volume in relation to the total volume of the second solvent system.

8. Method according to claim 6 or 7, wherein the second solvent system is constituted with C₂-C₄ alcohols, and notably ethanol, n-propanol, isopropanol, butanol, in particular n-butanol, MeTHF, or mixtures thereof.

9. Method according to any one of claims 6 to 8, wherein the second solvent system comprises a content in a solvent selected from C₂-C₄ alcohols, and notably ethanol, n-propanol, isopropanol, butanol, in particular n-butanol, and MeTHF, of at least 50%, notably at least 75%, in particular at least 90%, more particularly at least 95%, even more particularly at least 99% by volume in relation to the total volume of the second solvent system.

10. Method according to any one of claims 1 to 9, wherein the vegetable oil, oil from a microorganism or vegetable butter is selected from oil of soya, quinoa, rapeseed, corn, sunflower, sesame, lupin, cotton, coconut, olive, palm leaf, wheat germ, alfalfa, avocado, palm kernel, groundnut, copra, flax, castor bean, grape seeds, squash seeds, blackcurrant seeds, melon seeds, tomato seeds, pumpkin seeds, almond, hazelnut, nut, evening primrose, borage, safflower, false flax, oil poppy, macroalgae, microalgae such as *Chlorella,* and/or microorganisms, notably salt water, fresh water or terrestrial microorganisms, in particular yeasts, molds and, more particularly, bacteria, and mixtures thereof, and cocoa butter, illipe butter, shea butter, and mixtures thereof.

11. Method according to any one of claims 1 to 10, wherein the first solvent system consists of a *tert-*butyl ether, notably 2-ethoxy-2-methylpropane, also called ethyl-*tert*-butyl-ether (ETBE).

12. Method according to any one of claims 1 to 10, wherein the first solvent system consists of a first solvent selected from fluorinated aromatic solvents, notably trifluorotoluene (BTF) and hexafluorobenzene (BHF), solvents comprising at least one silicon atom, notably hexamethyldisiloxane (HMDS) and tetramethylsilane (TMS) or methyl-tetrahydrofuran (MeTHF), and a second solvent, not identical to the first, selected from tert-butyl ethers, notably 2-ethoxy-2-methylpropane, also called ethyl-tert-butyl-ether (ETBE), and 2-methoxy-2-methylpropane, also called methyl tert-butyl ether (MTBE).

13. Method according to any one of claims 1 to 10, wherein the first solvent system consists of one or more solvents selected from HMDS, HFB and BTF and one or more solvents selected from MTBE and ETBE.

14. Method according to any one of claims 1 to 10, wherein the first solvent system consists of ETBE.

15. Method according to any one of claims 1 to 10, wherein the unsaponifiable obtained is selected from a soya unsaponifiable, an avocado unsaponifiable, notably a furan fraction-rich avocado unsaponifiable and/or a sterol fraction-rich avocado unsaponifiable, and more particularly a mixture of avocado and soya unsaponifiables (ASU).
